Europäisches Patentamt

European Patent Office

Office européen des brevets

(19)

(11) Publication number: **0 185 809**
A1

## (12) EUROPEAN PATENT APPLICATION

(21) Application number: **84308712.3**

(22) Date of filing: **14.12.84**

(51) Int. Cl.⁴: **A 61 F 5/44**

(43) Date of publication of application:
**02.07.86 Bulletin 86/27**

(84) Designated Contracting States:
**AT BE CH DE FR GB IT LI LU NL SE**

(71) Applicant: **Manfredi, Frank Anthony**
**2026 West 95th Street**
**ClevelandOhio 44102(US)**

(72) Inventor: **Manfredi, Frank Anthony**
**2026 West 95th Street**
**ClevelandOhio 44102(US)**

(74) Representative: **Paget, Hugh Charles Edward et al,**
**MEWBURN ELLIS & CO. 2/3 Cursitor Street**
**London EC4A 1BQ(GB)**

(54) Urinary drain system.

(57) A male urinary collection and drain system includes a penis sheath (10), a flexible, accordion-like outlet tube (14) connecting the distal end of the sheath (10) to a urine collecting bag (12) via a positive-acting check valve (16) which prevents reverse flow, and a flexible, accordion-like drain tube (18) connected to the lower end of the bag and fitted with a manually operable valve (20). The bag is located at thigh level and is supported in that position primarily by waist-belt (22) and one or more suspension straps (24), with additional stability being provided by one or more leg-encircling straps (26).

The sheath includes one or more elastic O-rings (104) formed integrally with its side wall, preferably at the bottoms of peripheral grooves (102), for forming a seal with the penis and for holding the sheath in place.

EP 0 185 809 A1

./...

This invention relates to urinary collection and drain devices for individuals, male and female, who are partially or totally urinary incontinent.

## BACKGROUND

A variety of        urinary collection and drain devices have been disclosed in the prior art.  The basic purpose of such devices is to temporarily collect urine in a receptacle carried by the wearer and to provide for periodic draining of the urine from the receptacle through a manually operated valve into a toilet.  When the wearer is more or less physically active, i.e. ambulatory or confined partially or wholly  to a wheel chair, it is important that the device be comfortable, accommodate itself to the movements of the wearer and be easily drained, all without introducing blockage or backup of urine, leakage of urine while in place, and spillage during draining.

The devices which are commercially available and/or disclosed in the prior art serve the basic desired purpose but they tend to suffer from one or more disadvantages in the areas just mentioned.  Examples of prior art devices or components thereof especially for male use are disclosed in United States Patents 3,336,926 3,586,041, 3,608,552, 3,631,857, 3,661,156, 3,721,243, 3,724,461, 3,739,783, 3,749,096, 3,788,324, 3,835,857, 3,916,90? 3,926,233, 3,998,228, 3,999,550, 4,022,213, 4,055,179, 4,073,295, 4,202,335, ·4,232,677, 4,343,316, 4,344,432, 4,352,356 and 4,345,342,

## SUMMARY OF THE INVENTION

In accordance with the invention there is provided a urine collecting bag assembly for use in a urinary collecting and draining assembly comprising a bag having an upper end fitted with a urine inlet and a lower end fitted with a manually operable urine drain valve; an adjustable leg strap carried by the bag near its lower end for encircling the leg of a wearer; an adjustable waist strap for encircling the waist of the wearer; and at least one suspending strap having opposite ends connected to the waist strap and to the upper end of the bag, said suspending strap having a length appropriate to suspending the bag at a location on the upper leg of the wearer.

In accordance with the present invention in another aspect a male urinary collection and drain system includes a penis sheath of special novel construction , a flexible, accordion-like outlet tube connecting the distal end of the sheath to a urine collecting bag via a positive-acting check valve which prevents reverse flow, and a flexible, accordion- like drain tube connected to the lower end of the bag and fitted with a manually operable valve. The bag is located at thigh level and is supported in that position primarily by a waistbelt, with additional stability being provided by one or more leg straps. The sheath is of special construction in that it includes one or more elastic O-rings formed integrally with its side wall, preferably at the bottoms of peripheral grooves, for forming a seal with the penis and for holding the sheath in place. In another embodiment the sheath is held in place by an assembly of waist band, pouch and leg straps, with the O-rings serving primarily a sealing function rather than a holding function. In this embodiment the sheath extends loosely through a hole in the pouch and includes a peripheral flange which lies between the pouch and the body of the wearer so as to position the sheath properly.

### BRIEF DESCRIPTION OF THE DRAWINGS

Figure 1 is a view of a urinary collection and draining system embodying the principles of the present invention;

Figures 2,3 and 4 are fragmentary views of tubing useful in making the connections shown in Figure 1;

Figures 5 and 6 are partial sectional views of two embodiments of check valves;

Figure 7 is a partial sectional view of a drain valve;

Figure 8 is a partial sectional view of another drain valve;

Figure 9 is a view on an enlarged scale, of the sheath of Figure 1;

Figure 10 is a sectional view taken on the line 10-10 of Figure 9;

Figure 11 is a view of a longitudinally pleated sheath;

Figure 12 is a sectional view taken on the line 12-12 of Figure 11;

Figure 13 is a view of another longitudinally pleated sheath;

Figure 14 is a sectional view taken on the line 14-14 of Figure 13;

Figure 15 is a perspective view of another draining system; and

Figure 16 is a sectional view taken on the line 16-16 of Figure 15.

-5-

Fig. 17 is a perspective view of parts of a female urinary drain system corresponding to the view of Fig. 15 and, like Fig. 15, not showing the collecting bag and drain tube;

Fig. 18 is a side view of the urine collecting device of the female drain system of Fig. 17; and

Fig. 19 is a perspective view of the device of Fig. 18, shown in its expanded form.

## DETAILED DESCRIPTION

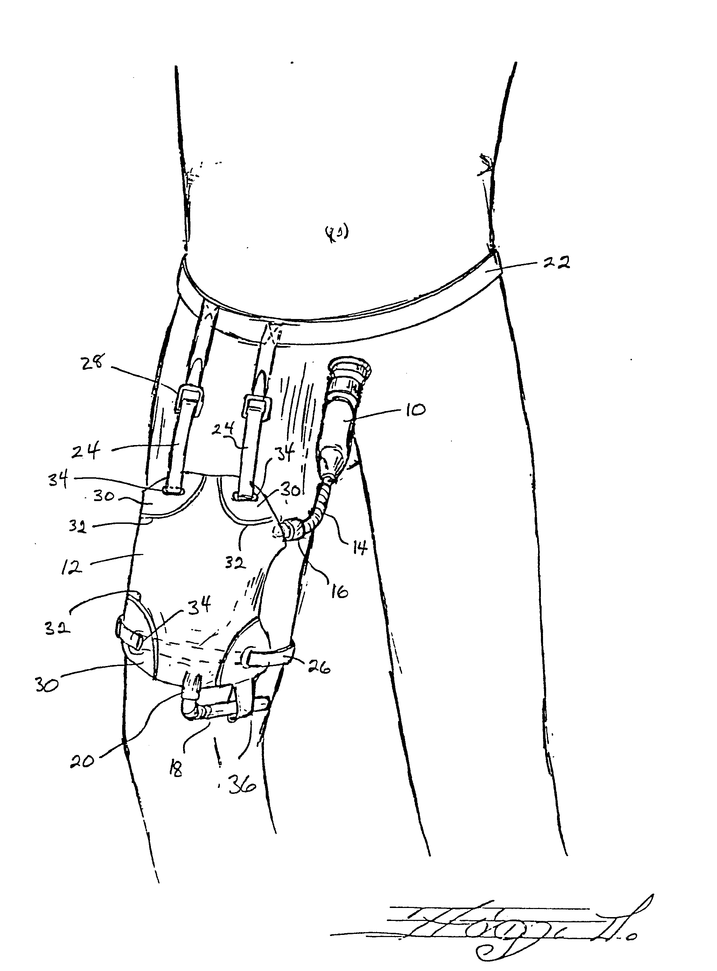

Figure 1 illustrates a urinary collection and drain system which embodies several features of the invention. The major parts of the system are a penis sheath 10 or condom, a collection receptacle or bag 12, a flexible bag-inlet conduit 14 connecting the distal end of the sheath with the bag 12 via a check valve 16 which prevents back flow of urine, and a short flexible drain tube 18 connected to the lower end of the bag 12 and fitted with a manually operable drain valve 20.

As is conventional the vertical dimension of the bag 12 is somewhat greater than its horizontal dimension so that the bag 12 will lie against the front surface of one of the wearer's upper legs and at the same time have an appropriate volume. The bag 12 is thus generally rectangular, although its corners may be considerably rounded to give a generally elliptical shape. In any case the urine inlet conduit 14 connects with the bag at a location essentially at a corner (or equivalent) of the bag 12 so that the length of the conduit 14 can be as short as possible and contain as few bends as possible. This is important because it lessens the chance that the conduit 14 will become kinked during body movements and thereby block the flow of urine into the bag 12. Backup of urine into the sheath and into contact with the penis should of course be avoided in order to prevent leakage of urine and the possibility of infection.

The bag 12 must remain in essentially a fixed position because if it shifts, the conduit 14 may become kinked,
collapsed or pulled loose. In the past it has been conventional
to support the weight of the bag 12 by two vertically spaced-
apart straps which encircle the upper leg of the wearer. This
arrangement has now been found to be often unsatisfactory because either it permitted the bag to shift vertically or laterally
or, if the straps were tightened enough to prevent shifting, the
straps were binding and uncomfortable on the leg. Accordingly,
in the system of the present invention the weight of the bag 12
is supported essentially wholly by an adjustable waist band 22
which encircles the waist of the wearer. Preferably the waist band
22 is quite narrow, for example, 1/2 inch to 1 inch. The bag 12
is suspended from the waist band 22 by one or more adjustable
suspension straps 24 so as to be located on the front surface of
the wearer's upper leg. Near the lower end of the bag 12 is
an adjustable stabilizing strap 26 which encircles the wearer's
leg. The straps 22, 24 and 26 are preferably of woven fabric which
is elastic in the longitudinal dimension of the respective strap,
and suitable buckles 28 or other adjusting means are included.

Conveniently the bag 12 is formed of soft flexible
plastics material such as polyvinyl chloride, by heat-sealing
the edges of two superimposed sheets of the plastic material.
In the illustrated embodiment each corner area 30 of the bag 12
is strengthened and sealed from the interior of the bag 12 by
an arcuate heat-sealed band 32. The area 30 can then be provided with one or more slots 34 through which the elastic straps are
threaded, thereby avoiding the use of hard or bulky connections
between the bag and the straps.

At the lower end of the bag 12 is a depending retaining
loop 36 for loosely holding the flexible drain tube 18 in a
convenient storage position, such as parallel to the lower edge
of the bag 12. As illustrated in Figure 1 the loop 36 is a
permanently closed loop formed by a heat sealing operation.
Alternatively the loop 36 can be formed as a strap folded over on
itself with the end of the strap being releasably connected to
the remainder of the strap, as with short strips of Velcro fastenir
fabric attached to the strap.

The conduit 14 and the drain tube 18 are made of naturall
flexible material such as polyvinyl chloride. To enhance flexibility, prevent kinking and render the members 14 and 18 more
accommodating to movements of the wearer's body these members are
radially fluted, bellows-like for at least a portion of their
length. The flutes may be formed so as to provide flexibility
without providing any substantial axial elastic extensibility or
they may be formed to provide such extensibility or they may be
formed to provide both flexibility and extensibility. Figure 2
illustrates a tube construction which is particularly suitable
for providing resilient axial extensibility, the tube wall having
flaps 38 extending outwardly and at an acute angle to the axis
of the tube. Figures 3 and 4 illustrate tube constructions which are suitable for providing flexibility. Figure 3 shows closely-spaced sharp-edged pleats 40 set at an angle to the tube axis, and Figure 4 shows radially extending pleats 42 with rounded edges.

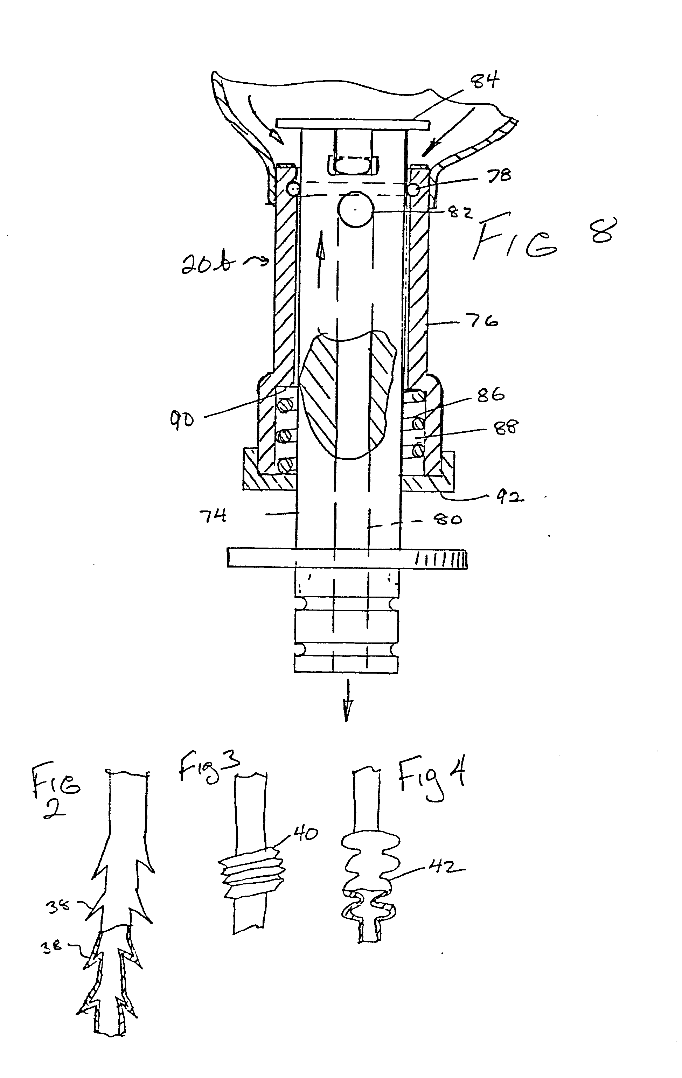

Figures 5 and 6 illustrate check valves 16a and 16b which are particularly suitable for use in the invention. The valve 16a includes a tubular piece 44 telescoped into a tubular piece 46 to the extent permitted by an external shoulder 48 on the piece 44. An internal cavity 50 is formed by and between the pieces 44 and 46 and residing in the cavity 50 is a floating closure disc 52. The diameter of the disc 52 is greater than the diameter of the bore in the piece 46 and smaller than the outer diameter of the end of the piece 44. The end of the piece 44 has a transverse groove 54 which permits flow in the direction of the arrow 56, i.e. into the bag 12. Reverse flow out of the bag 12 will force the disc 52 into sealing engagement with the bore in the piece 46 and thereby prevent flow into the conduit 14. The Figure 6 construction is similar to Figure 5 except that the closure member is a ball 58.

Figure 7 illustrates a particularly suitable drain valve 20a for use in the invention. As illustrated the valve is a two-piece assembly having an interiorly-threaded tubular part 60 and an externally-threaded tubular part 62 threaded into the piece 60. The inner end 64 of the part 62 is conical and is engageable with a complementary annular valve seat 66 on the part 60. The bore 68 of the piece 62 terminates in one or more transverse openings 70 located below the cone 64. The valve is shown in a closed position; slight unscrewing of the part 62 will disengage the cone 64 from the valve seat 66 and permit flow through the valve. It is preferred to connect the piece 60 to the bag 12 and the piece 62 to the drain tube 18, but this relationship can be reversed. The part 62 has a laterally projecting finger piece 72 which can be grasped by the fingers to aid in unscrewing the part 60. Only a slight twist, for exampl one-half turn, is necessary to open the valve.

Figure 8 illustrates another drain valve 20b. This valve comprises a tubular plunger axially slidable within a barrel 76, with an O-ring 78 forming a seal between the exterior of the plunger 74 and the interior of the barrel 76. The upper end of the barrel 76 is in communication with the bag 12. The bore 80 in the plunger 74 terminates in one or more transverse openings 82 located below the O-ring 78. The upper end of the plunger 74 carries a closure disc 84 the lower surface of which is engageabl

with the adjacent end of the barrel 76. A compression spring 86 is located in a cavity 88 formed by and between the plunger 74 and the barrel 76. The spring 86 surrounds the plunger 74 and bears against an internal shoulder 90 on the barrel 76 and a cap 92 on the barrel 76. The spring 86 normally biases the plunger 74 downwardly so that the closure disc 84 seals with the upper er of the barrel 76, preventing flow. When the plunger 74 is manua:

moved upwardly against the spring bias, the disc 84 moves away from the end of the barrel 76 and after further movement the aperture 82 passes above the O-ring 78, with the result that liquid will pass into the aperture 82 and downwardly through the bore 80.  The O-ring 78 can be carried in a peripheral groove in the plunger 74, and in such a construction flow through the valve will occur when the O-ring 78 rises above the end of the barrel.  The plunger 74 is illustrated in Figure 8 in an intermediate position.

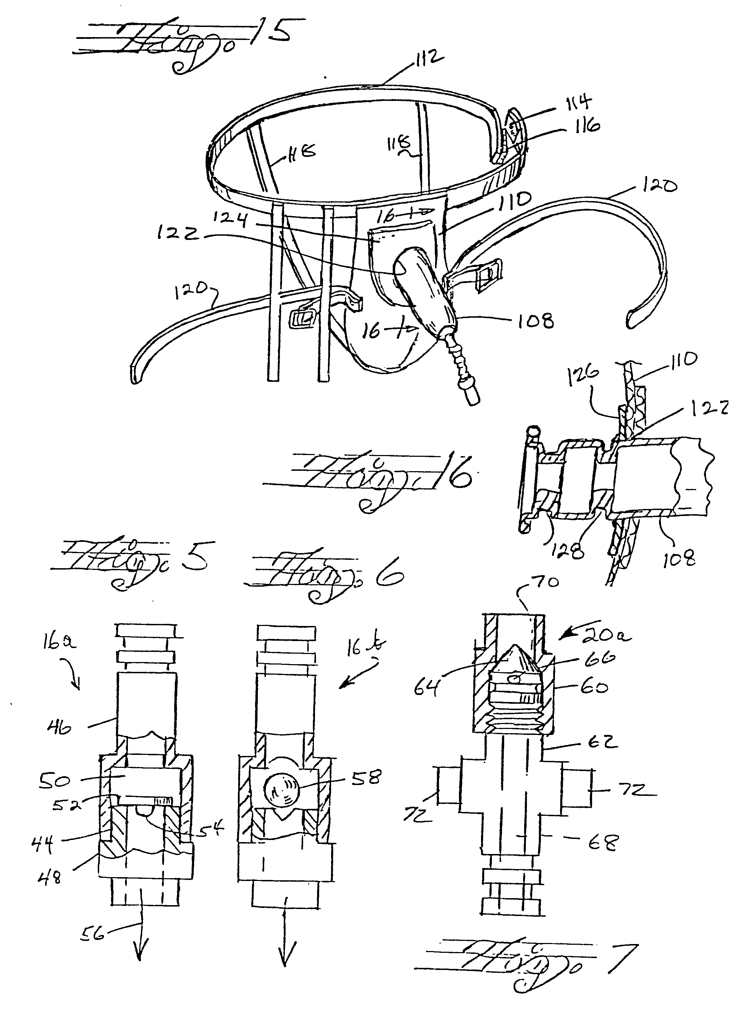

Figure 9 illustrates, on an enlarged scale, the penis sheath 10 of Figure 1.  The sheath is a tubular member molded of flexible elastic material such as rubber having an open proximal end 94 and a distal end 96 integral with a flexible conduit 98. The proximal end 94 has a conventional bead 100 to reinforce the sheath.  Axially spaced from the end 94 are two circumferential grooves 102 molded into the material of the sheath 10.  The bottom wall of each groove forms an integral O-ring 104 which forms a seal with the penis and also holds the sheath 10 in place.  To this end the bottom wall is of greater thickness than the side walls of the groove and the adjacent wall of the sheath.  With this construction the bottom wall, when radially expanded, produces a greater inward contracting force than do the axially adjacent portions of the sheath wall.  The connector tube 98 includes a bellows section 105 immediately adjacent the distal end of the sheath 10.

Figures 11 and 12 illustrate a penis sheath 10a which

0185809

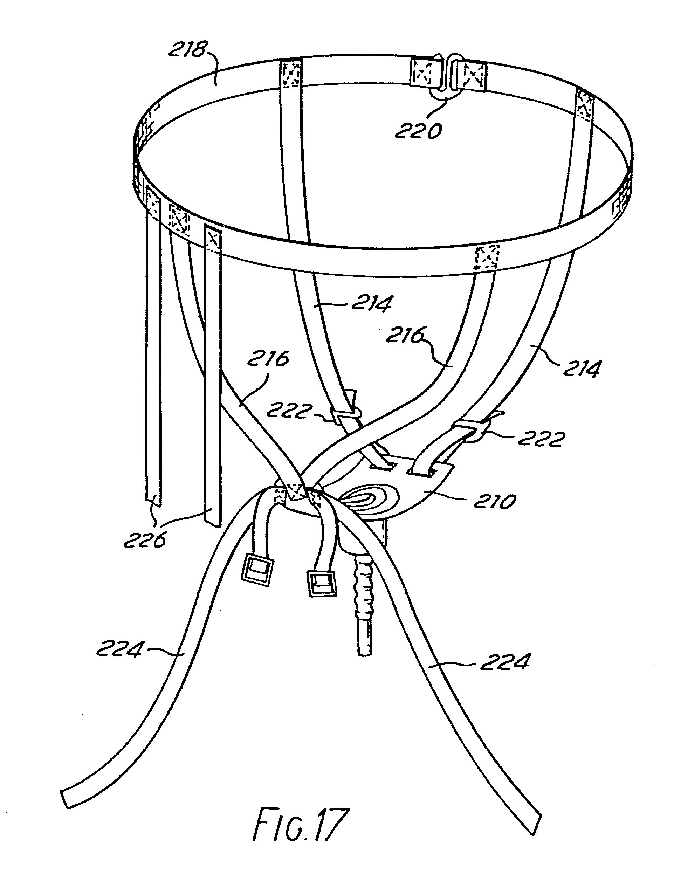

is similar to the sheath 10 of Figure 9 except that the sheath 10a is longitudinally pleated at 106 to allow greater radial extension. The pleats 106 are provided on the entire circumference. Figures 13 and 14 illustrate a similar sheath 10b having continuous fine pleats 107 entirely around the circumference of the sheath.

Figure 15 illustrates a draining system having a penis sheath 108 releasably carried by a fabric pouch 110 which is waist-supported. The upper end of the pouch is directly attached to a thin (e.g. 1/2 inch to 1 inch wide) elastic belt 112 having Velcro fasteners 114, 116 at its ends. The lower end of the pouch 110 is connected in a conventional manner to the rear part of the waist strap by elastic straps 118. Each side of the pouch 110 is provided with an adjustable strap 120 adapted to encircle one of the wearer's legs. The purpose of the straps 120 is to hold the pouch 110 snugly to the wearer's body in order to hold the removable sheath 108 in place, as described subsequently.

The front of the pouch 110 is provided with an aperture 122 around which a piece of reinforcing fabric 124 may be attached if necessary. The sheath 108 extends through the aperture 122. A radial flange 126 is fixed to the sheath near its proximal end and lies against the inner surface of the pouch 110 but is not attached thereto. The flange 126 is substantially stiffer than than the material of the sheath 108 and resiliently restrains or prevents the sheath 108 from being pulled out of the pouch 110.

The sheath 108 can, however, be easily removed in a rearward direction from the pouch 110 when the assembly is not being worn. When the assembly is being worn, with the leg straps 120 secured about the legs of the wearer, the flange 126 is pressed between the pouch 110 and the wearer's body and thereby stabilizes the position of the sheath 108. The sheath preferably includes molded-in O-rings 128 of the kind previously described. In this embodiment the O-rings 128 serve essentially only as urine seals, as the sheath is held in position by the straps.

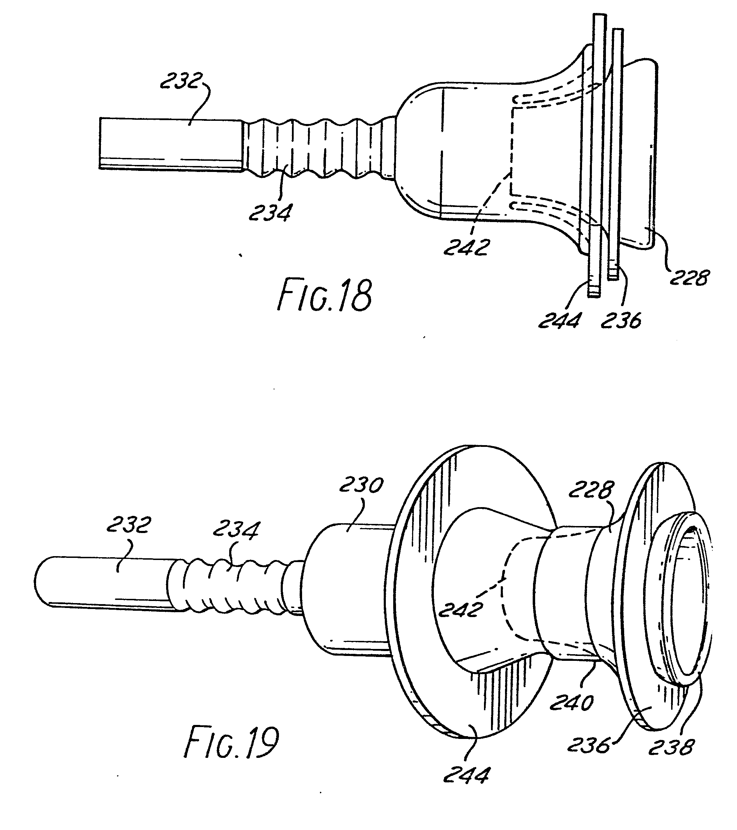

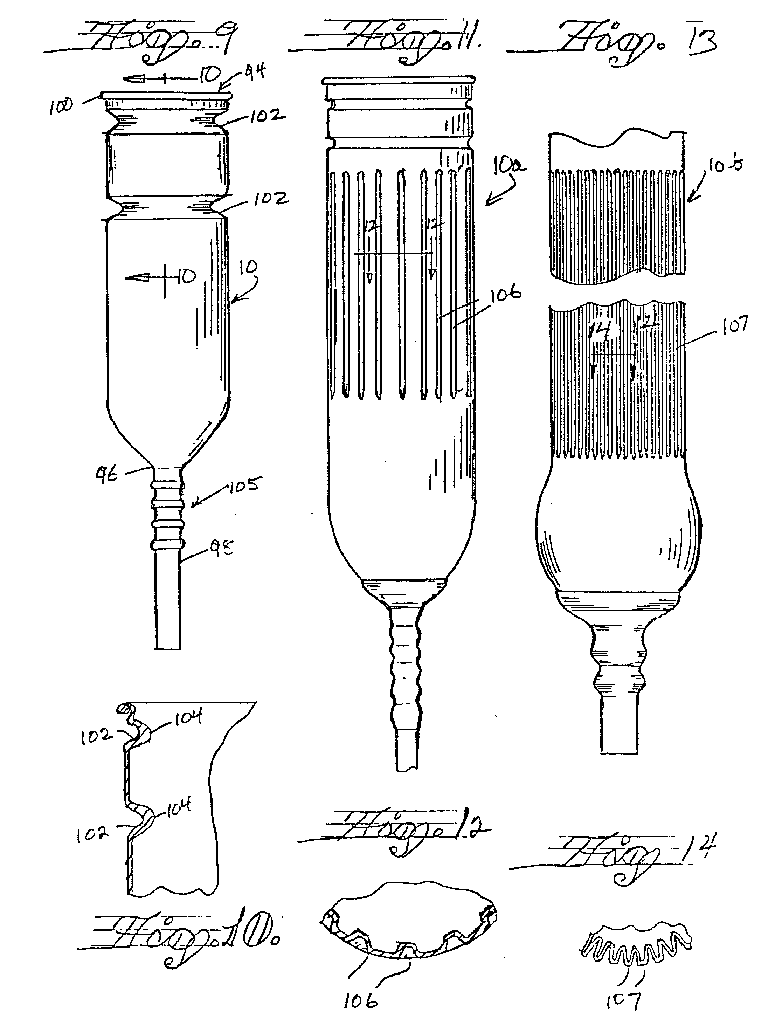

Figs. 17 to 19 show an adaptation of the drain system of Figs. 1 and 15 for use by a woman. Corresponding to the pouch 110 of the system shown in Fig. 15, that of Fig. 17 has an approximately square web 210 of soft textile fabric through a central hole of which is inserted the urine collection device 212 illustrated in Figs. 18 and 19. The web 210 is attached by four straps 214,216 to a resiliently extensible waist belt 218 which is here shown with a simple hook and loop connector 220 for its ends but which may alternatively be of adjustable length. The rear straps 214 are shown with optional length-adjustment buckles 222. Instead the front straps 216 may have length-adjustment means, or all four straps 214,216 may be adjustable in length.

As in the system of Fig. 15, also attached to the web 210 are two strip and buckle arrangements 224 for attaching around the upper legs of the wearer. By suitable attachment and adjustment of the belt and the straps, the web 210 can be made to lie comfortably against the crotch of the wearer.

Shown hanging from the belt 220 are two vertical straps 226 for attachment to the collecting bag, e.g. via buckles in the manner of the straps 24 showin in Fig. 1. The bag is suspended from the belt 220 by these straps 226, in use. In this embodiment the collecting bag, its drain tube and valve and its manner of support on the user's leg are exactly as shown in Fig. 1.

The collection device of Figs. 18 and 19 is in the form of a flanged funnel 228 of relatively thick, soft rubber, e.g. sponge rubber of a latex like material, attached to a flanged sleeve portion 230 formed of thin resilient flexible rubber sheet and integral with a narrower conduit portion 232 leading to the collecting bag via a check valve, like the conduit 14 of Fig. 1. Also, like the conduit 14 of Fig. 1, the conduit 232 is partly of bellows-like construction 234, to allow easy flexing and length variation without kinking or strain.

The funnel 228 is of sufficient rigidity to hold its shape approximately when in use without imposing discomfort on the user and has an exterior flange 236 slightly spaced from its oval open end 238, so that the funnel can be arranged by the user comfortably with the end 238 appropriately placed in relation to the urethra to collect the urine. The funnel 238 is attached to the end of the sleeve 230 at seal 240 closely behind the flange 236 but extends a substantialy distance further into the sleeve 230 to its distal end 242 which is freely located within the sleeve, so that when the sleeve and funnel are in their telescoped arrangement as shown in Fig. 18, the end 242 is spaced from the walls of the sleeve thereby inhibiting collection of liquid in this region and also flow of liquid back up the funnel 228. This reduces the risk of infection.

The sleeve 230 has a relatively rigid rubber flange 244 welded to its exterior (so that the interior of the sleeve 230 has no discontinuities which might collection urine). In use this flange 244 lies against the inner face of the web 210, and the flange 236 of the funnel lies against it. The funnel is thus held in place by the web 210.

CLAIMS

1.      A urinary drain assembly comprising a bag (12) having an upper end fitted with a urine inlet (14) and a lower end fitted with a manually operable urine drain valve (20); a leg strap (26) carried by the bag near its lower end for encircling the leg of a wearer; a waist strap (22;218) for encircling the waist of the wearer; and at least one suspending strap (24;226) having opposite ends connected to the waist strap and to the upper end of the bag, said suspending strap having a length appropriate to suspending the bag at a location on the upper leg of the wearer.

2.      A drain assembly according to claim 1 wherein said bag is generally rectangular or elliptical and further including, at one corner area, a urine inlet assembly (14) which includes a check-valve (16) for preventing flow out of said inlet assembly, and at the end opposite said inlet assembly a urine outlet assembly (18) which includes said manually operable valve and a flexible drain tube at least a portion of which is radially pleated in order to enhance bending without kinking.

3.      A drain assembly according to claim 1 or claim 2 for male use and further having a pouch (110) carried by the waist band, said pouch having an opening therein, a condom or penis sheath (108) extending loosely through the opening in the pouch, the sheath having a distal end provided with a urine outlet and a proximal open end; a radial flange (126) fixed on said sheath and located inside said pouch in engagement with the inner surface thereof, the flange being of greater diameter than the opening in said pouch to thereby restrain removal of said sheath from said pouch in an outward direction; and at least two leg straps (120) connected to said pouch for encircling the legs of the wearer.

4.      A drain assembly as in claim 3 wherein said sheath comprises a tubular member of molded flexible elastic material having, near but axially spaced from its proximal end, at least one circumferential molded-in groove (102) the bottom wall of which is thicker in radial dimension than the axially adjacent wall portions of the sheath, said thickened wall having a radially inwardly contracting force, when radially stretched, than the remainder of the sheath so as to function as an O-ring seal with the penis.

5.        A drain assembly according to claim 4 wherein said sheath comprises a tubular member of molded flexible elastic material having a plurality of longitudinally extending parallel pleats molded in the flexible elastic material.

6.        A urinary drain assembly according to claim 1 or claim 2 for female use and further having a web of material (210) suspended from the waist band (218) so as to be located at the crotch of the wearer, said web having an opening through it; a urine collection funnel (228) extending through the opening in the web and having a distal end forming an urine outlet and a proximal open end; a flange (236) fixed on said funnel and locatable so as to bear against the inside face of the web to retain the funnel in position; and at least two leg straps (224) connected to said web for encircling the legs of the wearer.

7.        A male urinary drain assembly comprising a waist band (22) for encircling the waist of the wearer; a pouch (110) carried by the waist band, said pouch having an opening therein; a condom or penis sheath (108) extending loosely through the opening in the pouch, the sheath having a distal end provided with a urine outlet and a proximal open end; a radial flange (126) fixed on said sheath and located inside said pouch in engagement with the inner surface thereof, the flange being of greater diameter than the opening in said pouch to thereby restrain removal of said sheath from said pouch in an outward direction; and at least two leg straps (120) connected to said pouch for encircling the legs of the wearer.

8.      A condom or penis sheath for use in a urinary drain assembly comprising a tubular member (10) of molded flexible elastic material, said member having an open proximal end and a distal end having connected thereto a flexible urine discharge conduit (98) of lesser diameter than the tubular member; said tubular member having, near but spaced axially from its proximal end, at least one elastic circumferential ring molded (104) integrally with the material of said sheath, said ring being radially outwardly stretchable and having a radially inwardly contracting force greater than the remainder of .said sheath so as to function in use as an O-ring which holds the sheath to the penis and forms a seal therewith.

9.      A condom or penis sheath for use in a urinary drain assembly comprising a tubular member (10a;10b) of molded flexible elastic material, said member having an open proximal end and a distal end having connected thereto a flexible urine discharge conduit (98) of lesser diameter than the tubular member; said tubular member having a plurality of longitudinally extending parallel pleats (106;107) molded in the flexible elastic material.

10. A female urinary drain assembly comprising a waist band (218) for encircling the waist of the wearer; a web (210) of material suspended from the waist band so as to be located at the crotch of the wearer, said web having an opening through it; a urine collection funnel (228) extending through the opening in the web and having a distal end forming an urine outlet and a proximal open end; a flange (236) fixed on said funnel and locatable so as to bear against the inside face of the web to retain the funnel in position; and at least two leg straps (224) connected to said web for encircling the legs of the wearer.

FIG 8

FIG 2

Fig 3

Fig 4

Fig. 15

Fig. 16

Fig. 5

Fig. 6

Fig. 7

FIG.17

232

234

242

228

244 236

FIG.18

232 234 230 228

242

240

236 238

244

FIG.19

6/6

0185809

Fig. 9

Fig. 11.

Fig. 13

Fig. 10.

Fig. 12

Fig. 14

*European Patent Office*

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| X | US-A- 2 133 130 (F. BUCHSTEIN) | | A 61 F 5/44 |
| | * Figure 1 * | 1 | |
| | --- | | |
| X | FR-A- 2 422 388 (J. FLESCHER) | | |
| | * Figures 1,2 * | 1 | |
| A | | 2,3 | |
| | --- | | |
| X | US-A- 2 628 617 (J.F. WRIGHT) | | |
| | * Figure 1 * | 1 | |
| A | | 3 | |
| | --- | | |
| X | US-A- 3 651 810 (G.E. ORMEROD) | | |
| | * Whole document * | 1,6 | TECHNICAL FIELDS SEARCHED (Int Cl 4) |
| Y | | 2-5 | |
| | --- | | A 61 F |
| Y | DE-A- 2 043 198 (H.H. BIJKERK) | | |
| | * Figure 1; page 8, lines 1-23; page 10, lines 5-13 * | 2 | |
| | --- | | |
| Y | GB-A- 871 862 (J. JOYNER et al.) | | |
| | * Page 2, lines 75-111; figures 1,2 * | 3-5 | |
| | --- | | |
| Y | GB-A- 637 978 (J. JOYNER et al.) | | |
| | * Page 3, lines 12-51; figures 1,2 * | 4,5 | |
| | --- | ./. | |

The present search report has been drawn up for all claims

| Place of search | Date of completion of the search | Examiner |
|---|---|---|
| The Hague | 14-08-1985 | WOLF |

## CLAIMS INCURRING FEES

The present European patent application comprised at the time of filing more than ten claims

☐ All claims fees have been paid within the prescribed time limit The present European search report has been drawn up for all claims

☐ Only part of the claims fees have been paid within the prescribed time limit The present European search report has been drawn up for the first ten claims and for those claims for which claims fees have been paid.

namely claims

☐ No claims fees have been paid within the prescribed time limit The present European search report has been drawn up for the first ten claims

## X | LACK OF UNITY OF INVENTION

The Search Division considers that the present European patent application does not comply with the requirement of unity of invention and relates to several inventions or groups of inventions.

namely
1) Claims 1-6: Urinary drain assembly

2) Claim 7: Means to retain a drain assembly in a male garment

3) Claim 8: Molded o-ring for a condom

4) Claim 9: Longitudinally pleated condom

5) Claim 10: Means to retain a drain assembly in a female garment

☐ All further search fees have been paid within the fixed time limit The present European search report has been drawn up for all claims

☐ Only part of the further search fees have been paid within the fixed time limit The present European search report has been drawn up for those parts of the European patent application which relate to the inventions in respect of which search fees have been paid.

namely claims

X None of the further search fees has been paid within the fixed time limit The present European search report has been drawn up for those parts of the European patent application which relate to the invention first mentioned in the claims

namely claims   1-6

European Patent
Office

**EUROPEAN SEARCH REPORT**

**0185809**
Application number

EP 84 30 8712

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| Y | US-A- 3 421 507 (V.D. GRESHAM)<br><br>* Column 2, lines 14-25; figure 2 * | 5 | |
| A | US-A- 4 000 649 (P. HANIFL)<br><br>* Abstract * | 2 | |
| A | WO-A- 81 01 957 (DAJEM)<br><br>* Abstract * | 3 | |
| A | US-A- 3 742 953 (J.W. LEE)<br><br>* Abstract * | 2,5 | |
| A | GB-A- 1 274 374 (SALT & SON)<br><br>* Figures 1,3 * | 3,5 | · TECHNICAL FIELDS SEARCHED (Int Cl 4) |

The present search report has been drawn up for all claims

| Place of search | Date of completion of the search | Examiner |
|---|---|---|
| | | |